# EUROPEAN PATENT APPLICATION

(11) **EP 4 838 538 A2**
(43) Date of publication of application: **11.11.2026**
(21) Application number: 26208318.1
(22) Date of filing: 20.01.2023
(51) Int. Cl.: G16H 40/60

(54) **USER INTERFACE DISPLAYS FOR ASSISTING IN MEDICAMENT BOLUS CALCULATION**

(30) Priority: 21.01.2022 US 202263301741 P
(62) Document number(s) of the earlier application(s) in accordance with Art. 76 EPC: 23152654.2 / 4 216 226
(71) Applicant: Insulet Corporation, Acton, MA 01720 (US)
(72) Inventor: LEE, Joon Bok, Acton (US); ZHENG, Yibin, Hartland (US); O'CONNOR, Jason, Acton (US); GALLAGHER, Kerrie, Wakefield (US); PHILBRICK, Lindsay, Sudbury (US); NGUYEN, Alex, Dallas (US); WILLIAMS, Joshua, Marlborough (US)
(74) Representative: Peterreins Schley

(57) **Abstract**

Exemplary embodiments may provide a user interface and logic for assisting a user in calculating a proper medicament bolus dosage. The user interface may be simple and easy to understand. The user interface may clearly specify needed inputs, such as a carbohydrates amount for a meal that is about to be ingested and the current glucose level reading for the user. Entered inputs may be displayed on the user interface. The user interface may depict key calculated values resulting from calculations that involve the inputs, including the total bolus dosage calculation. The user interface also may depict the values that contribute to the total bolus dosage calculation. Some of the input values, such as carbohydrates amount, may be prepopulated by the management device or medicament delivery device. The user interface may permit overriding of prepopulated input values.

## Description

### BACKGROUND

Conventional insulin delivery devices may deliver insulin to a user in different fashions. First, conventional insulin delivery devices may deliver basal insulin deliveries to the user. The basal insulin deliveries are delivered to the user on an on-going basis at regular intervals to help regulate glucose levels of the user. Second, conventional insulin delivery devices may deliver insulin boluses to the user upon demand. The insulin boluses typically are delivered to compensate for carbohydrates that are consumed in meals that may raise glucose levels or to correct high glucose levels. Insulin bolus dosages tend to be larger than basal delivery dosages and are not delivered as frequently.

Calculating the proper dosage for an insulin bolus may be a daunting challenge for a user. The user may need to calculate the bolus dosage from a current glucose value, an estimate of carbohydrates intake, insulin on board (IOB) and a glucose target for the user. The user may not properly estimate the amount of carbohydrates in a meal, the user may not know the current IOB, the user may not account for how much basal insulin is to be delivered after the insulin bolus is delivered and/or the user simply may rely on a rule of thumb, such as always setting the bolus dosage at 1 unit of insulin, that is not well suited for their current insulin needs.

To aid the user in determining a proper insulin bolus dosage, some conventional insulin delivery devices may provide insulin bolus dosage recommendations where software estimates an insulin bolus dosage for the user based on user input. Unfortunately, such conventional software may be confusing to the user or difficult to use. In some conventional software, multiple screens are displayed to the user to gather the requisite information to calculate the recommended bolus dosage. Such screens are often cluttered and confusing. Many screens may contain complex equations that are difficult for the user to understand. With these screens, the burden is on the user to calculate the amount of carbohydrates to be consumed. The user may estimate the amount of carbohydrates incorrectly or may not know how to determine the amount of carbohydrates for the upcoming meal.

### SUMMARY

In accordance with a first inventive aspect, an electronic device includes a display for displaying information to a user. The electronic device also includes a storage medium for storing data and computer programming instructions and a processor for executing the computer programming instructions. The computer programming instructions cause the processor to exhibit a user interface for calculating a dosage of a medicament for delivery by a medicament delivery device to the user on the display. The user interface includes an element for entering an amount of carbohydrates to be ingested by the user, an element for entering a current glucose level reading, an element for specifying a calculated total bolus dosage, and an element for specifying a medicament on board adjustment value, which displays how much the medicament already delivered to the user will compensate for the carbohydrates to be ingested. The computer programming instructions also cause the processor to exhibit the medicament on board adjustment value on the display in the element for specifying a medicament on board adjustment value, based on entry of a value in the element for entering an amount of carbohydrates to be ingested by the user or entry of a value in the element for entering a current glucose level reading.

The medicament may be at least one of insulin, a glucagon-like peptide receptor-1 (GLP-1) agonist, or pramlintide, for example. The user interface may include an element for displaying an adjustment to the bolus dosage due to a glucose level trend of the user. The computer programing instructions may cause the processor to calculate the adjustment to the bolus dosage due to the glucose level trend of the user. The computer programing instructions may cause the processor to calculate the medicament on board adjustment value. The element for specifying the calculated total bolus dosage may be editable to adjust the calculated total bolus dosage. The element for entering a current glucose level reading may be activatable to retrieve the current glucose level reading. The electronic device may be one of the medicament delivery device, a controller device for the medicament delivery device, a computing device or a wearable electronic device.

In accordance with another inventive aspect, an electronic device includes a display for displaying information to a user and a storage medium for storing data and computer programming instructions. The electronic device also includes a processor for executing the computer programming instructions. The computer programming instructions cause the processor to exhibit a user interface for calculating a dosage of a medicament for delivery by a medicament delivery device to the user on the display. The user interface includes an element for entering an amount of carbohydrates to be ingested by the user, and an element for specifying a calculated total bolus dosage. The computer programming instructions cause the processor to calculate an estimate of the amount of carbohydrates to be ingested by the user from data of past amounts of carbohydrate ingestion by the user and to prepopulate the element for entering the amount of carbohydrates to be ingested by the user with the calculated estimate of the amount of carbohydrates to be ingested by the user.

The calculating of the estimate of the amount of carbohydrates to be ingested by the user may include calculating an average of the past amounts of carbohydrate ingestion by the user. The calculating of an average of the past amounts of carbohydrate ingestion by the user may entail calculating an average of only ones of the past amounts of carbohydrate ingestion by the user that are for a like time of day to a current time of day. Each past amount of carbohydrate ingestion by the user may be associated with a type of meal, and the method may include identifying a type of a current meal to be ingested. The calculating of the average of the past amounts of carbohydrates ingestion by the user may include calculating an average of only ones of the past amounts of carbohydrate ingestion by the user that are for a like type of meal as the current meal to be ingested. The type of meal may be one of breakfast, lunch, dinner, or snack. The computer programming instructions may further cause the processor to visually distinguish the prepopulated calculated estimate of the amount of carbohydrates to be ingested by the user from an amount of carbohydrates to be ingested that has been entered by the user via the element for entering the amount of carbohydrates to be ingested by the user.

In accordance with a further inventive aspect, a method is performed by a processor of an electronic device. The method includes estimating with the processor a quantity of a medicament that will be delivered by an automated medicament delivery device over an upcoming range of time during a current day by calculating an average quantity of medicament delivered to the user by the automated medicament delivery device for the range of time on past days. The method also includes displaying on a display the estimated quantity as part of a user interface for specifying a bolus dosage of the medicament to be delivered by the automated medicament delivery device to assist the user.

The estimated quantity may be a range of quantities. The estimating may entail estimating a minimum quantity of medicament that will be delivered by the automated medicament delivery device over the upcoming range of time during the current day and estimating a maximum quantity of medicament that will be delivered by the automated medicament delivery device over the upcoming range of time during the current day. The minimum quantity and the maximum quantity may constitute the boundaries of the range of quantities. The minimum quantity of medicament may be estimated based at least in part on latest glucose level reading, target glucose level, correction factor for the user and medicament on board. The minimum quantity of medicament may be estimated based at least in part on latest glucose level reading, target glucose level, medicament on board and basal medicament deliveries to be delivered over the range of time. The medicament may be at least one of insulin, a glucagon-like peptide receptor-1 (GLP-1) agonist, or pramlintide, for example.

### BRIEF DESCRIPTION OF THE DRAWINGS

Figure 1 depicts an illustrative medicament delivery system suitable for use in exemplary embodiments.
Figure 2 depicts an illustrative display screen for specifying an insulin bolus dosage with no carbohydrates amount or glucose value specified in an exemplary embodiment.
Figure 3 depicts an illustrative display screen for specifying an insulin bolus dosage with a carbohydrates quantity but no current glucose level specified in an exemplary embodiment.
Figure 4 depicts a flowchart of illustrative steps that may be performed in exemplary embodiments when a carbohydrates quantity alone is entered by a user.
Figure 5 depicts an illustrative display screen where a carbohydrates amount and a current glucose level have been entered in an exemplary embodiment.
Figure 6 depicts a flowchart of illustrative steps that may be performed in exemplary embodiments when a current glucose level and a carbohydrates amount are entered by a user.
Figure 7 depicts an illustrative display screen where a current glucose level has been entered in an exemplary embodiment.
Figure 8 depicts a flowchart of illustrative steps that may be performed in exemplary embodiments when only a current glucose level is entered by a user.
Figure 9 depicts an illustrative display screen where a carbohydrates amount, a current glucose level and a user adjustment to the bolus dosage have been entered in an exemplary embodiment.
Figure 10 depicts a flowchart of illustrative steps that may be performed in exemplary embodiments when an adjustment amount for the bolus dosage is entered by the user.
Figure 11 depicts an illustrative display screen where a user directly enters the bolus amount in an exemplary embodiment.
Figure 12 depicts a flowchart of illustrative steps that may be performed in exemplary embodiments to activate a glucose level user interface element on a display screen to retrieve a current glucose level reading.
Figure 13 depicts illustrative display screens where an estimate of carbohydrates amount is prepopulated and then overridden in an exemplary embodiment.
Figure 14 depicts a flowchart of illustrative steps that may be performed in exemplary embodiments to prepopulate the carbohydrates amount user interface element and update the element when a user overrides the estimate.
Figure 15 depicts an illustrative display screen showing areas that may be activated to clear the carbohydrates amount user interface element or the meal bolus amount user interface element.
Figure 16 depicts a flowchart of illustrative steps that may be performed in exemplary embodiments to override a populated value on a display screen.
Figure 17 depicts a flowchart of illustrative steps that may be performed in exemplary embodiments to estimate an average amount of carbohydrates consumed per meal.
Figure 18 depicts a flowchart of illustrative steps that may be performed in exemplary embodiments to estimate an average amount of carbohydrates consumed per a period around meals.
Figure 19 depicts a flowchart of illustrative steps that may be performed in exemplary embodiments to calculate and show estimated insulin delivery amounts for a period following an insulin bolus delivery.
Figure 20 depicts an illustrative user interface showing estimated insulin delivery amounts for a period following an insulin bolus delivery.
Figure 21 depicts a flowchart of illustrative steps that may be performed in exemplary embodiments to calculate a minimum estimated insulin delivery amount for a period following an insulin bolus delivery.
Figure 22 depicts a flowchart of illustrative steps that may be performed in exemplary embodiments to calculate a maximum estimated insulin delivery amount for a period following an insulin bolus delivery.

### DETAILED DESCRIPTION

Exemplary embodiments described herein may provide a user interface and logic for assisting a user in calculating a proper medicament bolus dosage. The user interface may be simple and easy to understand. The user interface may clearly specify needed inputs, such as a carbohydrates amount for a meal that is about to be ingested and the current glucose level reading for the user. Entered inputs may be displayed on the user interface. The user interface may depict key calculated values resulting from calculations that involve the inputs, including the total bolus dosage calculation. The user interface also may depict the values that contribute to the total bolus dosage calculation.

The user interface may be shown on a management device for a medicament delivery device. Alternatively, the user interface may be shown on a display of the medicament delivery device or other devices, such as a smartwatch, fitness monitor, or another type of wearable device. The user interface may also be displayed on a remote display, e.g., a vehicle infotainment display screen. These devices individually or in combination may perform the requisite calculations to display certain values on the display.

The user interface may be contained in a single display screen. The user interface may be simple and uncluttered. The user interface may be progressive such that as input values are provided, the user interface is updated in real time or near real time to display calculated values that contribute to the total medicament bolus calculation. Each of the calculated values (whether positive or negative) that contribute to the total medicament may be summed to produce the total medicament bolus calculation.

Some of the input values, such as carbohydrates amount, may be prepopulated by the management device or medicament delivery device. The prepopulated carbohydrates amount may be calculated from past carbohydrates amounts of previously ingested meals and/or past bolus amounts. The current glucose level input may also be prepopulated from a recently obtained stored value obtained from a sensor or may be obtained on demand from the sensor when the user interface is requested. The user interface may also include features that enable a user to request obtaining input values. For example, the user interface may enable a user to take an action, such as a swipe, to obtain the most recent glucose level reading from the sensor.

The user interface may permit overriding of prepopulated input values. For example, the carbohydrates input value may be prepopulated and then overridden by the user. The prepopulated value may be visually distinguished to identify the value as a prepopulated value that may be overridden. The total medicament bolus value may be overridden by the user entering their own value or by entering an adjustment value. The user also may have the option of not entering a carbohydrates amount or a glucose level value and instead, just entering a total bolus amount.

The exemplary embodiments may also provide a user interface that informs the user of the amount of medicament that is anticipated to be delivered after delivery of the bolus over a time window, such as 90 minutes or another time period. For instance, the medicament delivery device may be programmed to deliver basal medicament dosages over the time window, and it is useful to know a range of possible delivery amounts that may be delivered over the time window. The exemplary embodiments may look at variables such as current glucose level, IOB, correction factor, etc. to estimate the anticipated delivery amounts. Since the anticipate delivery amounts are not fixed but rather are dependent on variables and user behavior, the exemplary embodiments may display a minimum amount and a maximum amount to define a range of amounts that are possible. This enables the user to better appreciate how much medicament may be delivered in addition to the bolus. The information helps the user to consider yet another variable that should be considered in calculating the total bolus dosage.

The exemplary embodiments provide a user interface and logic for assisting in calculating meal boluses, correction boluses and boluses that are a combination of meal boluses and correction boluses. The user may rely solely on the logic to calculate the bolus amount or may override portions of the calculation using the user interface. The user interface also permits the user to not rely at all on the logic to calculate the total bolus amount but rather enables the user to enter a custom bolus amount.

The exemplary embodiments will be described below. Many of the example implementations will be described relative to the medicament being insulin. Nevertheless, it should be appreciated that other medicaments may be used.

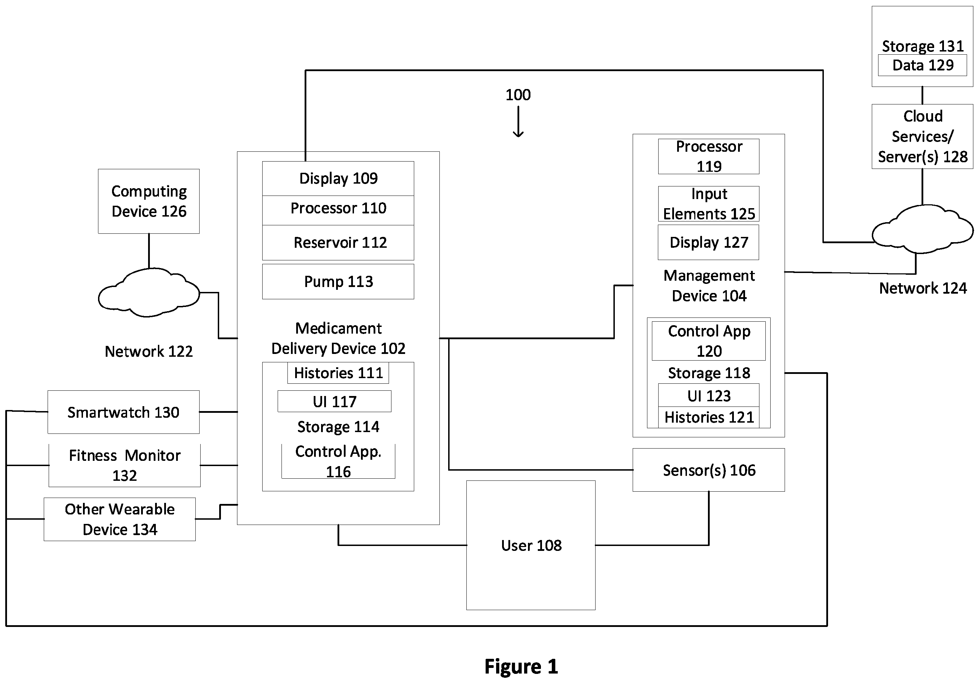

Figure 1 depicts an illustrative medicament delivery system 100 that is suitable for delivering a medicament to a user 108 in accordance with exemplary embodiments. The medicament delivery system 100 includes a medicament delivery device 102. The medicament delivery device 102 may be a wearable device that is worn on the body of the user 108 or carried by the user. The medicament delivery device 102 may be directly coupled to a user (e.g., directly attached to a body part and/or skin of the user 108 via an adhesive or the like) or carried by the user (e.g., on a belt or in a pocket) with the medicament delivery device 102 being connected to an infusion site where the medicament is injected using a needle and/or cannula. In a preferred embodiment, a surface of the medicament delivery device 102 may include an adhesive to facilitate attachment to the user 108.

The medicament delivery device 102 may include a processor 110. The processor 110 may be, for example, a microprocessor, a logic circuit, a field programmable gate array (FPGA), an application specific integrated circuit (ASIC) or a microcontroller. The processor 110 may maintain a date and time as well as other functions (e.g., calculations or the like). The processor 110 may be operable to execute a control application 116 encoded in computer programming instructions stored in the storage 114 that enables the processor 110 to direct operation of the medicament delivery device 102. The control application 116 may be a single program, multiple programs, modules, libraries or the like. The processor 110 also may execute computer programming instructions stored in the storage 114 for a user interface 117 that may include one or more display screens shown on display 109. The display 109 may display information to the user 108 and, in some instances, may receive input from the user 108, such as when the display 109 is a touchscreen.

The control application 116 may control delivery of a medicament to the user 108 per a control approach like that described herein. The storage 114 may hold histories 111 for a user, such as a history of basal deliveries, a history of bolus deliveries, and/or other histories, such as a meal event history, exercise event history, glucose level history and/or the like. In addition, the processor 110 may be operable to receive data or information. The storage 114 may include both primary memory and secondary memory. The storage 114 may include random access memory (RAM), read only memory (ROM), optical storage, magnetic storage, removable storage media, solid state storage or the like.

The medicament delivery device 102 may include one or more housings for housing its various components including a pump 113, a power source (not shown), and a reservoir 112 for storing a medicament for delivery to the user 108. A fluid path to the user 108 may be provided, and the medicament delivery device 102 may expel the medicament from the reservoir 112 to deliver the medicament to the user 108 using the pump 113 via the fluid path. The fluid path may, for example, include tubing coupling the medicament delivery device 102 to the user 108 (e.g., tubing coupling a cannula to the reservoir 112), and may include a conduit to a separate infusion site.

There may be one or more communications links with one or more devices physically separated from the medicament delivery device 102 including, for example, a management device 104 of the user and/or a caregiver of the user, a sensor 106, a smartwatch 130, a fitness monitor 132 and/or another variety of wearable device 134. The communication links may include any wired or wireless communication links operating according to any known communications protocol or standard, such as Bluetooth^{®}, Wi-Fi, a near-field communication standard, a cellular standard, or any other wireless protocol.

The medicament delivery device 102 may interface with a network 122 via a wired or wireless communications link. The network 122 may include a local area network (LAN), a wide area network (WAN) or a combination therein. A computing device 126 may be interfaced with the network, and the computing device may communicate with the medicament delivery device 102.

The medicament delivery system 100 may include one or more sensor(s) 106 for sensing the levels of one or more analytes. The sensor(s) 106 may be coupled to the user 108 by, for example, adhesive or the like and may provide information or data on one or more medical conditions and/or physical attributes of the user 108. The sensor(s) 106 may be physically separate from the medicament delivery device 102 or may be an integrated component thereof.

The medicament delivery system 100 may or may not also include a management device 104. In some embodiments, no management device is needed as the medicament delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as processor, a micro-controller, or the like. The management device 104 may be used to program or adjust operation of the medicament delivery device 102 and/or the sensors 106. The management device 104 may be any portable electronic device including, for example, a dedicated device, a smartphone, a smartwatch, or a tablet. In the depicted example, the management device 104 may include a processor 119 and a storage 118. The processor 119 may execute processes to manage a user's glucose levels and to control the delivery of the medicament to the user 108. The medicament delivery device 102 may provide data from the sensors 106 and other data to the management device 104. The data may be stored in the storage 118. The processor 119 may also be operable to execute programming code stored in the storage 118. For example, the storage may be operable to store one or more control applications 120 for execution by the processor 119. The one or more control applications 120 may be responsible for controlling the medicament delivery device 102, such as by controlling the AID delivery of insulin to the user 108. The storage 118 may store the one or more control applications 120, histories 121 like those described above for the medicament delivery device 102, and other data and/or programs.

A display 127, such as a touchscreen, may be provided for displaying information. The display may display user interface (UI) 123. The display 127 also may be used to receive input, such as when it is a touchscreen. The management device 104 may further include input elements 125, such as a keyboard, button, knobs, or the like, for receiving input form the user 108.

The management device 104 may interface with a network 124, such as a LAN or WAN or combination of such networks via wired or wireless communication links. The management device 104 may communicate over network 124 with one or more servers or cloud services 128. Data, such as sensor values, may be sent, in some embodiments, for storage and processing from the medicament delivery device 102 directly to the cloud services/server(s) 128 or instead from the management device 104 to the cloud services/server(s) 128. The cloud services/server(s) 128 may provide output from the model 115 as needed to the management device 104 and/or medicament delivery device 102 during operation.

Other devices, like smartwatch 130, fitness monitor 132 and wearable device 134 may be part of the medicament delivery system 100. These devices 130, 132 and 134 may communicate with the medicament delivery device 102 and/or management device 104 to receive information and/or issue commands to the medicament delivery device 102. These devices 130, 132 and 134 may execute computer programming instructions to perform some of the control functions otherwise performed by processor 110 or processor 119, such as via control applications 116 and 120. These devices 130, 132 and 134 may include displays for displaying information. The displays may show a user interface for providing input by the user, such as to request a change or pause in dosage or to request, initiate, or confirm delivery of a bolus of a medicament, or for displaying output, such as a change in dosage (e.g., of a basal delivery amount) as determined by processor 110 or management device 104. These devices 130, 132 and 134 may also have wireless communication connections with the sensor 106 to directly receive analyte measurement data.

A wide variety of medicaments may be delivered by the medicament delivery device 102. The medicament may be insulin for treating diabetes. The medicament may be glucagon for raising a user's glucose level. The medicament may also be a glucagon-like peptide (GLP)-1 receptor agonists for lowering glucose or slowing gastric emptying, thereby delaying spikes in glucose after a meal.

The functionality described below for the exemplary embodiments may be under the control of or performed by the control application 116 of the medicament delivery device 102 or the control application 120 of the management device 104. In some embodiments, the functionality may be under the control of or performed by the cloud services or servers 128, the computing device 126 or by the other enumerated devices, including smartwatch 130, fitness monitor 132 or another wearable device 134.

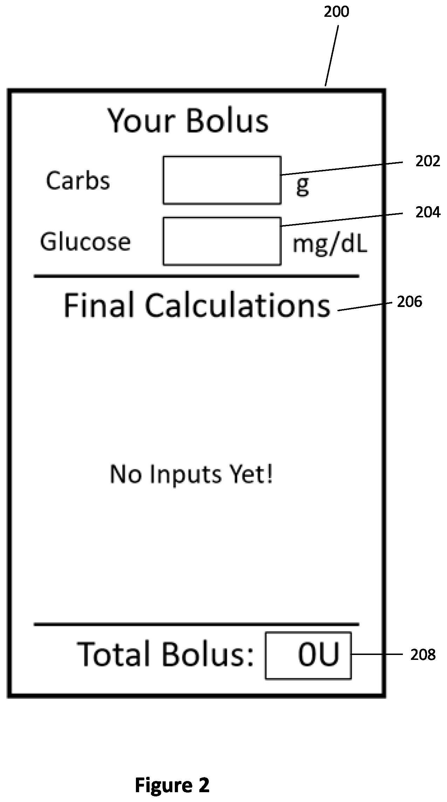

Figure 2 depicts an illustrative display screen 200 of an exemplary embodiment for assisting a user 108 in calculating a proper bolus dosage for an example where the medicament is insulin. The display screen 200 may be, for instance, part of the user interface 117 shown on display 109 or the user interface 123 displayed on display 127. The display screen 200 is purposefully minimalist to not be visually cluttered. In addition, the display screen 200 is designed to be simple and easily understood by the user 108. The display screen 200 includes a text box 202 for entering the amount of carbohydrates that the user 108 expects to ingest in the meal for which the insulin bolus is intended to compensate. The display screen 200 also includes a text box 204 for entering a most recent glucose level value for the user 108. The display screen 200 includes a final calculations section 206 in which calculations for determining the input bolus dosage based on the input values entered in text boxes 202 and/or 204 are shown. Lastly, text box 208 displays the calculated total insulin bolus dosage. Since no carbohydrates amount and no glucose level value have been entered yet via text boxes 202 or 204, text box 208 shows a 0 units total insulin bolus dosage and no calculations are shown in the final calculations section 206.

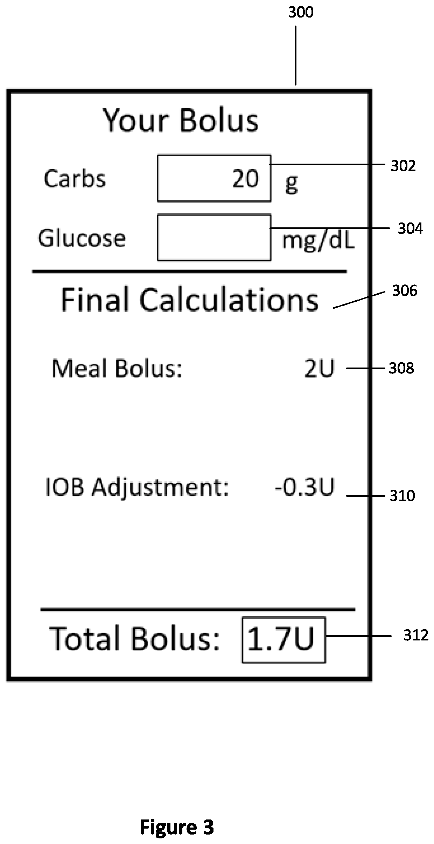

The display screen 200 of Figure 2 may be progressive in that as inputs are entered or retrieved, the display screen 200 is updated in response. The display screen 200 may be live in that it is updated in real time or near real time. Figure 3 depicts an updated display screen 300 in which a carbohydrates amount has been entered into text box 302. In this example, a value of 20 grams has been entered into text box 302. As can be seen in Figure 3, the final calculations section 306 has been updated. Specifically, a meal bolus amount 308 is displayed. The amount shown of two units of insulin is based upon the carbohydrates amount entered in text box 302. The 20 grams of carbohydrates warrant an insulin bolus of 2 units of insulin to compensate for the carbohydrates. The final calculations section 306 has also been updated to display an IOB adjustment value 310. The IOB adjustment value 310 is an adjustment in the insulin bolus dosage to compensate for the IOB of the user 108. The IOB represents the insulin already delivered to the user 108 that still has the potential to reduce the glucose level of the user 108. Since the IOB may compensate for a portion of the glucose rise resulting from the meal that is to be ingested, the medicament delivery system 100 of the exemplary embodiments accounts for the IOB so that the total insulin bolus dosage is not too large.

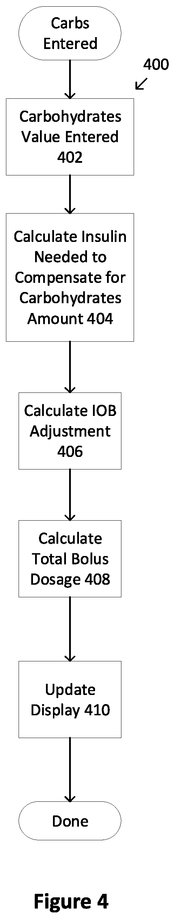

Figure 4 depicts a flowchart 400 of illustrative steps that may be performed to produce the updated screen display 300 of Figure 3. At 402, a carbohydrates amount value is entered, such as by the user 108 entering a value in text box 302. At 404, control application 116 or 120 calculates the amount of insulin needed to compensate for the entered carbohydrates amount. The insulin to carbohydrate ratio (ICR) for the user 108 may be used to determine the amount of insulin needed. At 406, an IOB adjustment is calculated. Specifically, the current IOB for the user is examined, and a determination is made as to how much the IOB will decrease the glucose level of the user 108 to help compensate for the carbohydrates to be ingested. At 408, the total insulin bolus dosage is calculated by subtracting the IOB adjustment that was calculated at 406 from the insulin needed to compensate for the carbohydrates amount that was calculated at 404. Thus, for example, in Figure 3, an IOB adjustment amount of 0.3 units is subtracted from the meal bolus amount of 2.0 units to produce a total bolus amount of 1.7 units. At 410, the display screen 300 is updated to reflect the relevant values, such as the total bolus amount 312, the meal bolus amount 308 and the IOB adjustment amount 310. Hence, the user 108 is given a clear and concise picture of how the total insulin bolus amount was determined.

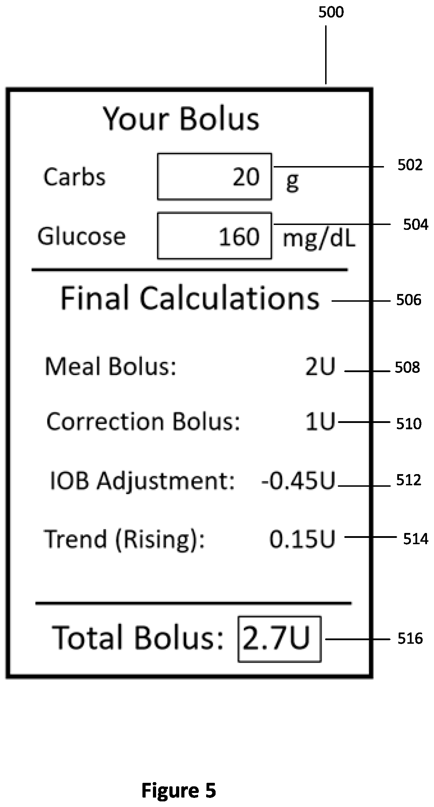

Figure 5 depicts an illustrative display screen 500 that may be shown to the user 108 when both the carbohydrates amount value and a current glucose level value are entered. As can be seen in Figure 5, the user 108 has entered the carbohydrates amount value in text box 502 and a most recent glucose level value in text box 504. The final calculations section 506 is updated. The final calculations section 506 displays the meal bolus amount 508 and an IOB adjustment amount 512 but also displays additional information. A correction bolus amount 510 is displayed, and a glucose trend amount 514 is also displayed. The correction bolus amount is warranted because the glucose level in text box 504 is elevated. The correction bolus of one unit will help to reduce that glucose level. As to the glucose trend adjustment 514, the control application 116 or 120 looks at the current trend in glucose levels and based on the direction and magnitude of the trend, makes any necessary adjustments to help avoid poor glucose level management that otherwise might happen in view of the trend.

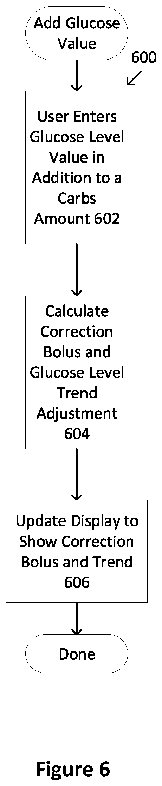

Figure 6 depicts a flowchart 600 of illustrative steps that may be performed in exemplary embodiments when both the carbohydrates amount and a current glucose level value have been entered by the user 108. At 602, the user enters the current glucose level value via text box 504. At 604, the control application 116 or 120 calculates a correction bolus value if needed based on the entered glucose level and a glucose level trend adjustment. The correction bolus value may be calculated when the current glucose level value exceeds the target glucose level value by over a threshold amount. The correction bolus can be calculated by determining how much the current glucose level value needs to be reduced via a correction bolus and applying the correction factor for the user 108 to determine how much insulin is needed to produce the desired reduction in glucose level. At 606, the screen display 500 is updated to show the correction bolus value 510 and the glucose trend adjustment value 512.

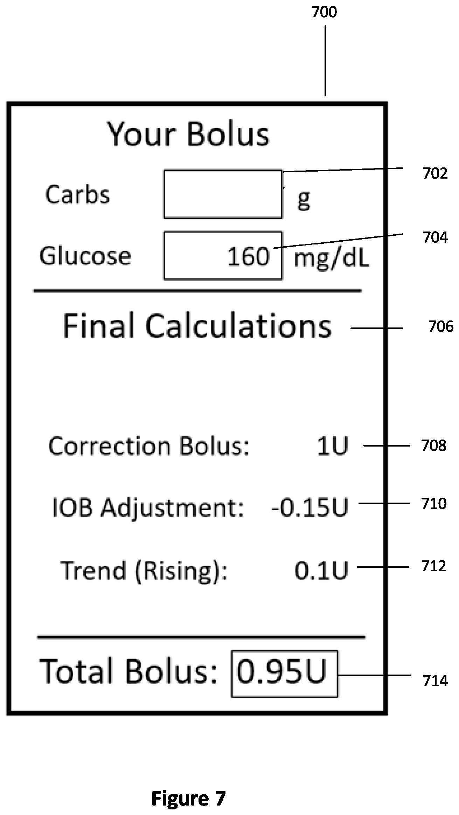

Figure 7 depicts a display screen 700 where the user 108 has not entered a carbohydrates amount but rather has only entered a current glucose level. In this instance, the user 108 is seeking to calculate the dosage amount for a correction bolus to reduce the current glucose level. Text box 702 remains empty, whereas text box 704 contains a current glucose level value of 160 mg/dL. In response to the entry of the current glucose level in text box 704, the final calculations section 706 displays a correction bolus amount 708 of 1.0 units of insulin and an IOB adjustment amount of 0.15 units of insulin. The final calculations section 706 also displays the glucose trend adjustment 712 of 0.1 units of insulin. The adding of the values 708, 710 and 712 produces the total bolus dosage shown in text box 714 of 0.95 units of insulin.

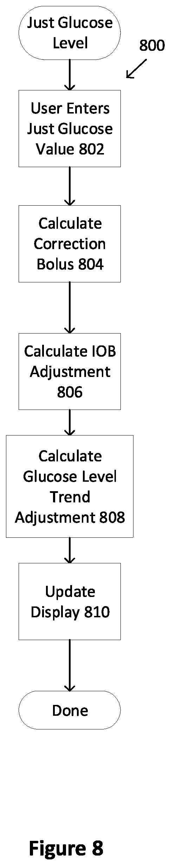

Figure 8 depicts a flowchart 800 of illustrative steps that may be performed in exemplary embodiments when just a glucose level value is entered by the user 108, such as depicted in Figure 7. At 802, the user 108 enters a current glucose value, such as in text box 704. No carbohydrates amount value is entered. At 804, the control application 116 or 120 calculates the correction bolus amount, such as was described above relative to Figure 4. In addition, at 806, the control application 116 or 120 calculates the IOB adjustment amount as has also been described above relative to Figure 4. At 808, the control application 116 or 120 calculates the glucose level trend adjustment 808 as described above relative to Figure 6. At 810, the display screen (such as display screen 700) is updated to show the correction bolus amount 708, the IOB adjustment amount 710, the trend adjustment amount 712 and the total insulin bolus amount 714.

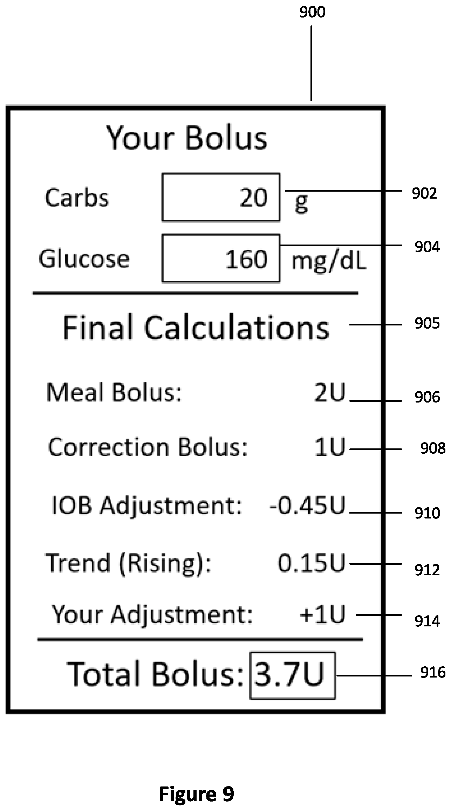

Figure 9 depicts the display screen 900 in which a user 108 has provided an adjustment amount for the calculated total bolus. A carbohydrates amount value has been entered in text box 902 and a glucose level value has been entered in text box 904. Responsive to these entries, the final calculations section 905 has been updated to show a meal bolus 906, a correction bolus 908, an IOB adjustment 910, a glucose trend adjustment 912 and a total bolus amount in text box 916. The final calculations section 905 also shows the adjustment amount of the user 914. The display screen 900 is like the display screen 500 shown in Figure 5 but for the adjustment amount of the user 914 and the resulting change in the total bolus amount shown in text box 916. Since the user has entered an adjustment amount of 1 unit of insulin for the correction bolus amount 908, the total bolus amount shown in text box 916 is increased by 1 unit relative to the corresponding amount shown in text box 516 on display screen 500 of Figure 5. Thus, the exemplary embodiments enable the user to enter adjustments to the total calculated insulin bolus amount and to show the adjustment amount on the display screen 900 in a clear and uncomplicated fashion.

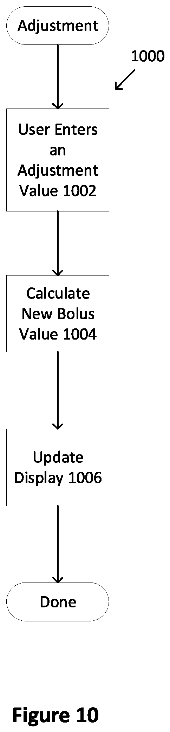

Figure 10 depicts a flowchart 1000 of illustrative steps that may be performed in exemplary embodiments in the instance in which a user 108 answers an adjustment amount. At 1002, the user 108 enters an adjustment value (see 914). The adjustment value is an adjustment, either positive or negative, relative to the calculated total bolus amount. At 1004, the control application 116 or 120 calculates the new total bolus dosage value in view of the adjustment provided by the user 108. At 1006, the display is updated accordingly to show the adjustment amount 908 and the calculated new total insulin bolus amount 916. In Figure 9, the new total insulin bolus amount is 3.7 units of insulin, as shown in text box 916.

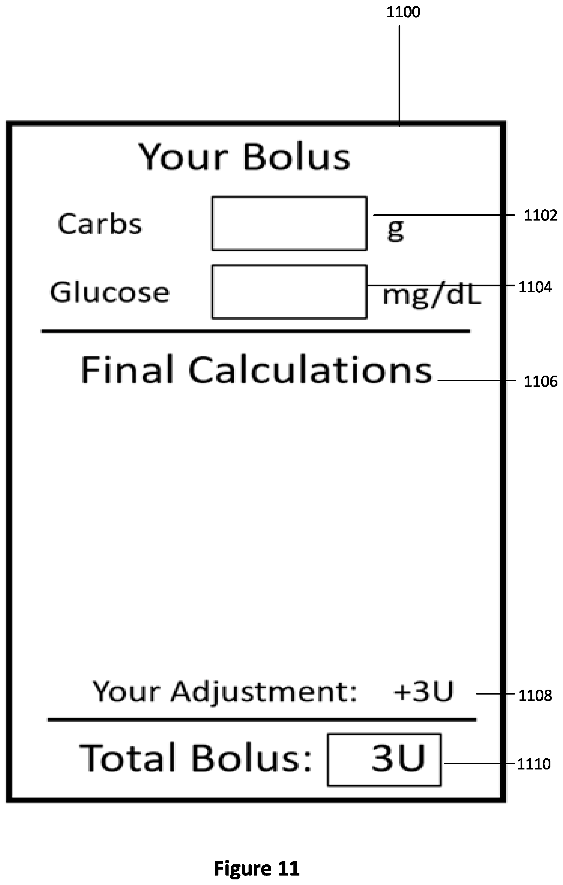

Figure 11 depicts another alternative display screen 1100 where the user 108 has not entered a carbohydrates amount value or a current glucose level value. Instead, the user 108 wishes to explicitly specify the dosage of the insulin bolus requested without any calculations by the control application 116 or 120. No values are entered in text box 1102 or text box 1104. The Final calculations section 1106 only shows the adjustment amount 1108 of 3 units of insulin entered by the user 108. The total insulin bolus amount is shown as 3 units in text box 1110.

From the above, it can be seen that the single use interface design accommodates meal boluses (see Figure 3 and 4), correction boluses (see Figures 7 and 8) and boluses that are for both correction and meals (see Figures 5 and 6). Moreover, the user can make adjustments to the total bolus values calculated by the control application 116 or 120 (see Figures 9 and 10). In addition, the user may rely on the same user interface screen display to specify an insulin bolus dosage without relying on calculations by the control application 116 or 120.

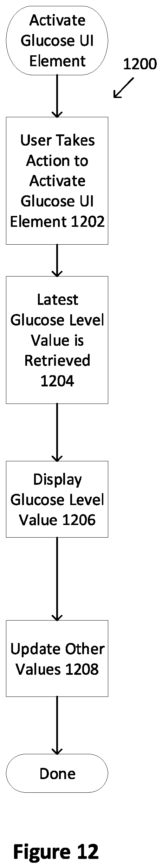

The exemplary embodiments need not rely on the user 108 alone to enter a current glucose level value; rather, in some instances, the control application 116 or 120 may retrieve a current glucose level value from storage 114 or 118 or may prompt a sensor 106 to provide such a current glucose level value. Figure 12 provides a flowchart 1200 of illustrative steps that may be performed in an exemplary embodiment where the control application 116 or 120 retrieves the current glucose level value. At 1202, the user 108 takes an action to activate the glucose user interface element. For example, the user 108 may swipe right over the text box 1104. At 1204, this action prompts the control application 116 or 120 to retrieve the latest glucose level value. At 1206, the glucose level value that was retrieved is displayed on the user interface, such as on a display screen. At 1208, the retrieved glucose level value is entered, and the other values on the display screen are updated accordingly as has been described above.

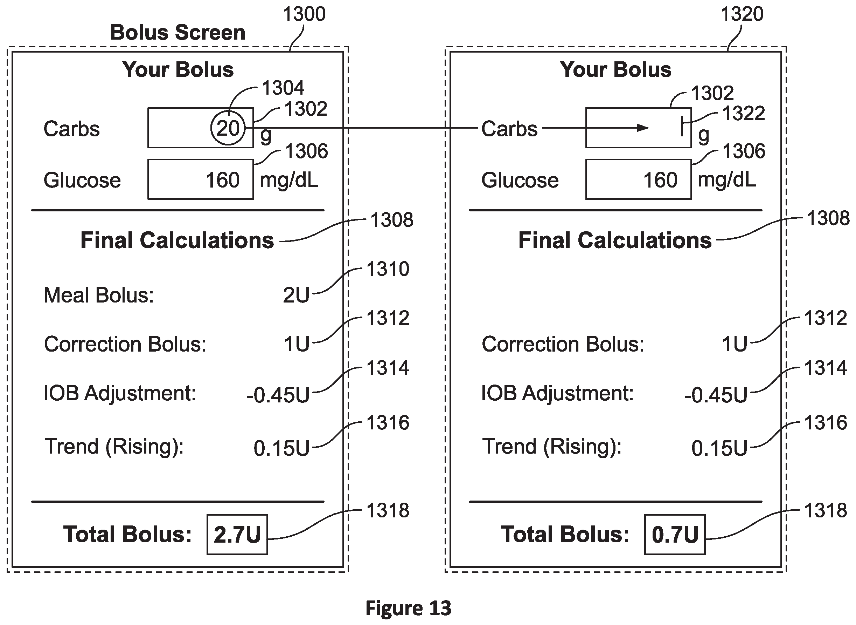

The exemplary embodiments may allow the user to override certain values that are populated by the control application 116 or 120. Figure 13 depicts a pair of display screens 1300 and 1320 that help to illustrate such overriding of populated values. In initial display screen 1300, text box 1302 has been populated with a value 1304 of 20 grams by the control application 116 or 120. The display screen 1300 also displays text box 1306, which displays a current glucose level value of 160. This display screen 1300 includes a final calculations section 1308 holding a meal bolus value 1310, a correction bolus value 1312, an IOB adjustment value 1314 and a glucose trend adjustment value 1316. These values 1310, 1312, 1314 and 1316 are all generated as described above due to the entry of the carbohydrates amount value in text box 1302 and the current glucose level value in text box 1306. A total bolus amount of 2.7 units of insulin is displayed in text box 1318. The carbohydrates amount value 1304 is visually distinguished to indicate that it is a prepopulated value. In this instance, the value 1304 is grayed out. It should be appreciated that other types of visual distinction, such as color, highlights, font choice, and the like, may be provided to identify the value is a prepopulated value. The user 108 may take an action, such as selecting the prepopulated value 1304 by positioning a cursor on the value 1304 and selecting the value 1304. In response, the display screen may change to be like that of display screen 1320 where there is no value in text box 1302 four the amount of carbohydrates. A cursor 1322 may be positioned in the text box 1302 to allow the user to enter an overriding value for the amount of carbohydrates. Since there is no carbohydrates value entered in text box 1302, the display 1320 is updated to no longer show the meal bolus 1310 and the total bolus amount is changed to be 0.7 units of insulin rather than 2.7 units of insulin in text box 1318.

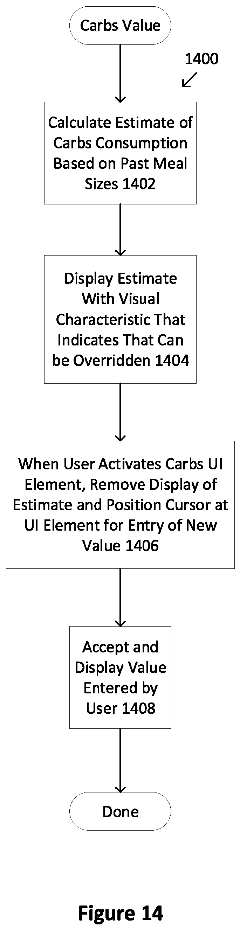

Figure 14 depicts a flowchart 1400 of illustrative steps that may be performed to prepopulate and then override the prepopulated carbohydrates amount value, such as in text box 1302 in Figure 13. At 1402, the control application 116 or 120 calculates an estimate of carbohydrates consumption based on past meal sizes. Thus, the control application 116 or 120 looks to the history data to determine the carbohydrates amounts of past meals and generates an estimate from the history data, such as an average carbohydrates amount for the past meals. At 1404, as was mentioned above, the calculated estimate may be displayed with a visual characteristic the indicates that the estimate may be overridden. At 1406, the user 108 activates the carbohydrates amount user interface element, such as text box 1302, and the control application 116 or 120 removes the display of the estimate and displays a cursor 1322 for entry of a new value. At 1408, when the user enters a value, the value is accepted and displayed on the screen display.

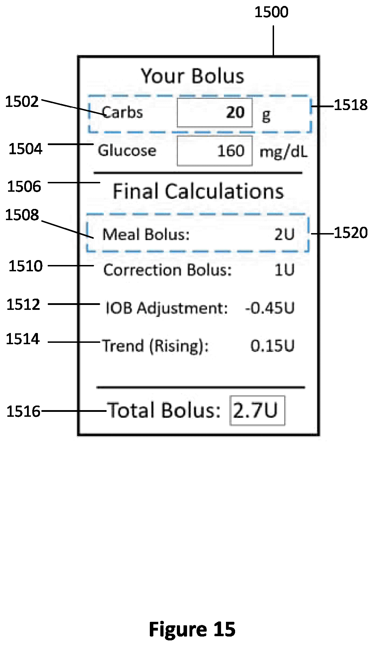

Figure 15 depicts a display screen 1500 that uses a different mechanism to override or clear input values that have already been entered. As shown in Figure 15, the display screen 1500 includes text boxes 1502 and 1504 for entering a carbohydrates amount and a current glucose level, respectively. Since these inputs have been provided in text boxes 1502 and 1504, the final calculations section 1506 includes a meal bolus amount 1508, a correction bolus amount 1510, an IOB adjustment amount 1512 and a glucose trend adjustment amount 1514. The display screen also includes total bolus amount 1516. The dotted boxes 1518 and 1520 indicate that the carbohydrates amount 1502 and the meal bolus amount 1508 may be cleared by swiping in the area bounded by the dotted boxes 1518 and 1520, respectively. The clearing removes the entered values 1502 or 1508 so that new values may be entered, or the entries may remain blank.

It should be appreciated that the swiping feature for dotted boxes 1518 and 1520 is intended to be merely illustrative. Other user interface mechanisms may be provided to clear values on the display screen and to permit overriding of values.

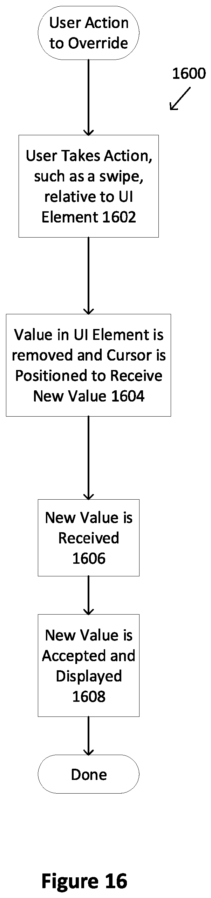

Figure 16 depicts a flowchart 1600 of illustrative steps that may be performed for a user 108 to override values. At 1602, the user takes a designated action, such as a swipe, relative to a UI element to clear the value associated with the UI element. At 1604, in response, the value is cleared from the UI element and the cursor is positioned to receive a new value. At 1606, the new value is received, such as by the user typing the new value. At 1608, the new value is accepted and displayed, such as in the UI element.

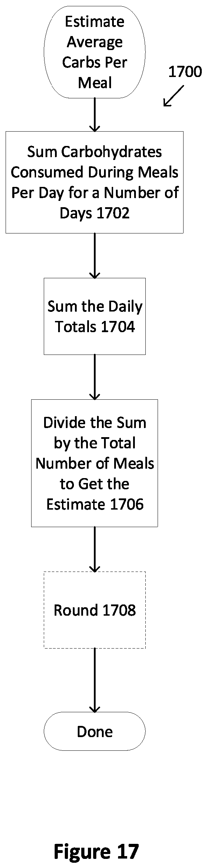

In order to prepopulate input values, the control application 116 or 120 must perform calculations. For example, to estimate the carbohydrates amount per meal, the control application 116 or 120 must perform calculations. A suitable equation for calculating an average of the user's previous carbohydrates amounts per meal is: ${m}_{rec} \left(j\right) = \frac{{\sum }_{j = 1}^{k} {\sum }_{i = 1}^{{n}_{meals} \left(k\right)} CHO \left(j, i\right)}{{\sum }_{j = 1}^{k} {n}_{meals} \left(j\right)}$ (Equation 1). where *m_{rec}*(*j*) is the average over *k* days, *j* is an index of days, *i* is an index of meals, *CHO*(*j*, *i*) is the carbohydrates amount in meal *i* on day *j*, and *n*ₘₑₐₗₛ() is the number of meals on an indexed day. This average may serve as the estimate of the carbohydrates amount per meal. Figure 17 provides a flowchart 1700 of illustrative steps that may be performed in calculating the average. At 1702, the amounts of carbohydrates consumed per day are summed for a number of days. At 1704, the daily totals are then summed. 1702 and 1704 are reflected in the following portion of equation 1: ${\sum }_{j = 1}^{k} {\sum }_{i = 1}^{{n}_{meals} \left(k\right)} CHO \left(j, i\right)$. At 1706, the sum of the daily totals is divided by the total number of meals (i.e., ${\sum }_{j = 1}^{k} {n}_{meals} \left(j\right)$) to get an estimate. Since the estimate may not need be expressed in very high resolutions units, such as 0.1 grams or even 0.5 grams, the value may be round down to the closest 5 grams increment at 1708. This rounding may be performed by applying the following equation: ${m}_{rec , final} \left(j\right) = 5 ⋅ floor \left(\frac{{m}_{rec} \left(j\right)}{5}\right)$ (Equation 2). The *floor()* function takes the integer portion of the quotient. The rounded value may be used as the estimate when rounding is applied.

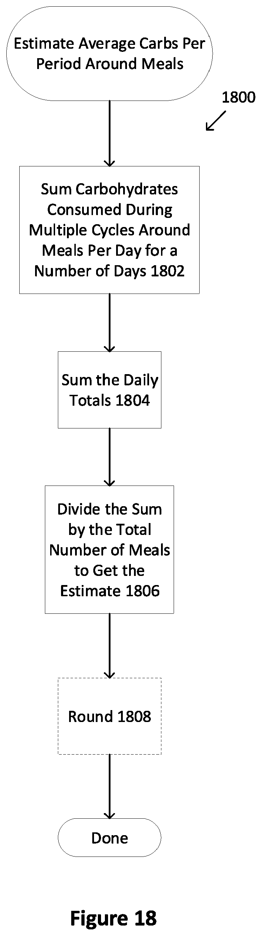

The user 108 may deliver multiple meal boluses in a short period of time for extended meals or meals where user may not finish a portion of the meals. In view of this user behavior, the estimate calculation may be modified to take a moving sum of at least three cycles (a cycle is a 5 minute interval in this instance). The modified estimate may be calculated as: ${m}_{rec} \left(j\right) = \frac{{\sum }_{j = 1}^{k} {\sum }_{i = 1}^{{n}_{meals} , 15 min \left(k\right)} {\sum }_{r = 1}^{3} CHO \left(j, i, r\right)}{{\sum }_{i = 1}^{k} {\sum }_{r = 1}^{3} {n}_{meals , 15 min} \left(i, r\right)}$ (Equation 3), where *r* is an index to the 3 cycles. Figure 18 depicts a flowchart of illustrative steps that may be performed to calculate this estimated average. At 1802, the amount of carbohydrates consumed during the three cycles per meal is summed for each day. At 1804, the daily totals are summed. Steps 1802 and 1804 are captured by ${\sum }_{j = 1}^{k} {\sum }_{i = 1}^{{n}_{meals} , 15 min \left(k\right)} {\sum }_{r = 1}^{3} CHO \left(j, i, r\right)$ in the above Equation 3. At 1806, the sum of the days is divided by the total number of meals over the days to get the estimate. At 1808, the resulting estimate optionally may be rounded down, as detailed above.

It should be appreciated that the estimate need not be for all meals; but rather there may be separate estimates for each meal. For example, there may be an estimate for breakfast, an estimate for lunch and an estimate for dinner. Such estimates may be calculated by determining the average of the respective meals separately. For example, only breakfast carbohydrates amounts would be summed over the days to generate a breakfast average. The designation of a meal as breakfast, lunch or dinner may be based on information provided by the user or by time periods that are typical for such meals. For instance, meals consumed between 7am to 11am may be presumed to breakfast, whereas meals consumed between 11am and 2pm may be presumed to be lunch, and meals consumed between 5pm and 9pm may be presumed to be dinner.

One challenge a user 108 may a face in calculating an insulin bolus dosage is what insulin is slated to be delivered in the future that may influence the calculation of a dosage for the insulin bolus. For example, basal insulin deliveries may be scheduled to be delivered automatically on an on-going basis and in most instances, will be delivered for some time after an insulin bolus is delivered to the user 108. Hence, it is useful to get an estimate of the amount that will be delivered over a period where the insulin bolus will affect the glucose level of the user, such as 90 minutes, following the delivery of the insulin bolus. Exemplary embodiments may calculate a range of possible insulin delivery amounts over the 90 minutes following delivery of the insulin bolus and may display the estimated range to the user 108.

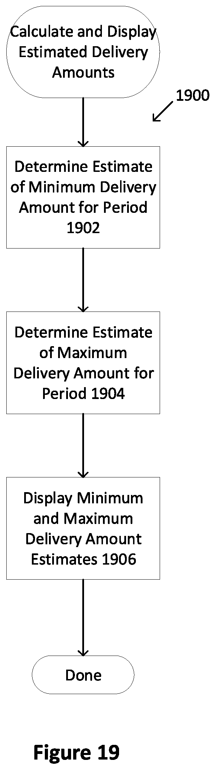

Figure 19 depicts a flowchart 1900 of illustrative steps that may be performed to calculate and display the estimated range for delivery amounts following the delivery of an insulin bolus. At 1902, an estimate of the minimum delivery amount for the specified period is determined. The particulars of how this estimate of the minimum delivery amount is determined will be provided below. At 1904, an estimate of the maximum delivery amount for the specified period is determined. The particulars of how this estimate of the maximum delivery amount is determined will be provided below. At 1906, the maximum and minimum estimates are displayed to the user 108.

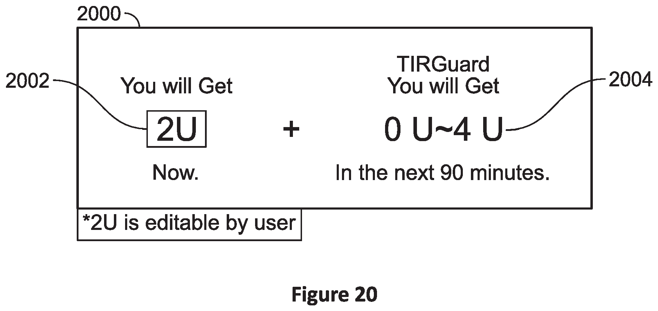

Figure 20 shows an illustrative UI element 2000 for displaying the range of estimates of amounts of insulin that will be delivered for the 90-minute period following delivery of an insulin bolus. The UI 2000 includes the magnitude of the dosage of the insulin bolus that is about to be delivered 2002 and the range 2004 of amounts that may be delivered over the next 90 minutes. In this example, between 0 and 4 units of insulin will be delivered over the next 90 minutes. The insulin bolus dosage may be editable directly from the UI element 2000.

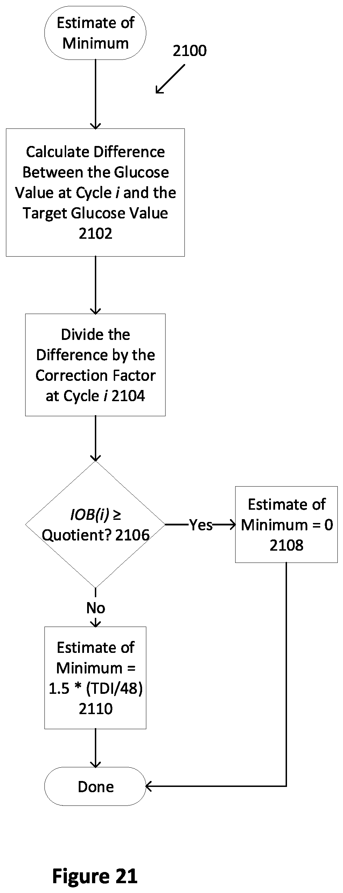

Figure 21 depicts a flowchart 2100 of illustrative steps that may be performed to determine the estimate of the minimum amount of insulin that will be delivered in the 90-minute period following the delivery of the insulin bolus. The following equation summarizes an illustrative approach to the determination of minimum estimate: ${I}_{est , min} \left(i\right) = \left\{\begin{matrix}0 & IOB \left(i\right) \geq \frac{G \left(i\right) - target \left(i\right)}{CF \left(i\right)} \\ \frac{TDI}{48} ∗ 1.5 & IOB \left(i\right) < \frac{G \left(i\right) - target \left(i\right)}{CF \left(i\right)}\end{matrix}\right.$ (Equation 4), where *I_{est,min}*(*i*) is the estimate of the minimum, *IOB*(*i*) is the insulin on board at cycle *I, G*(*i*) is the glucose level at cycle *I, target*(*i*) is the glucose level target at cycle *I, CF*(*i*) is the correction factor for the user 108 at cycle *I and TDI* is the total daily insulin for the user 108. At 2102, the difference between the glucose value at cycle *I* and the target glucose level at cycle *I* is determined (i.e., *G*(*i*) *- target*(*i*))*.* This difference indicates how much the current glucose level is over or under target. At 2104, the difference is divided by the correction factor of the user at cycle *I* (i.e., $\frac{G \left(i\right) - target \left(i\right)}{CF \left(i\right)}$). The resulting quotient, when positive, indicates how much insulin is needed to bring the glucose level of the user to target. At 2106, the quotient is compared to IOB for the user 108. At 2108, if IOB is greater than or equal to the quotient, no additional insulin is needed as the IOB provide sufficient insulin action. If not, at 2110, the estimate of the minimum is 1.5 x (*TDI*/48), which is the portion of TDI that is delivered in a 90-minute period.

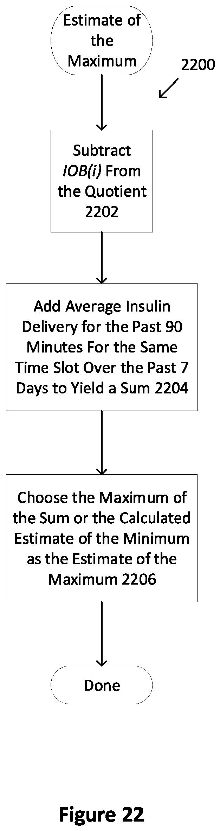

Figure 22 depicts a flowchart 2200 of illustrative steps that may be performed in an exemplary embodiment to determine an estimate of the maximum amount of insulin to be delivered to the user 108 in the 90 minutes following the delivery of an insulin bolus. The following equation summarizes the approach to determining the maximum: ${I}_{est , max} \left(i\right) = max \left({I}_{est , min},\frac{G \left(i\right) - target \left(i\right)}{CF \left(i\right)}-IOB\left(i\right)+\frac{{\sum }_{j = 1}^{7} {I}_{90} \left(j\right)}{7}\right)$ (Equation 5), where *I_{est,max}*(*i*) is the estimate of the maximum, max() is a function that outputs the maximum value among a set of values and *I*₉₀(*j*) is insulin delivered on day j in the same 90 minute time interval as the 90 minute interval following the current time, where ${I}_{90} \left(j\right) = {\sum }_{k = 288 ∗ j}^{288 ∗ j + 18} I \left(i-k\right)$ (Equation 6) given than there are 288 cycle per a day.

At 2202, to begin the process of calculating the maximum, *IOB(i)* is subtracted from the quotient $\frac{G \left(i\right) - target \left(i\right)}{CF \left(i\right)}$. At 2204, the resulting value is added to the insulin quantity delivered over the same 90-minute time interval for the past seven days (i.e., $\frac{{\sum }_{j = 1}^{7} {I}_{90} \left(j\right)}{7}$). At 2206, the greater of the sum or the estimated minimum is selected as the estimate of the maximum.

While exemplary embodiments have been described herein, it should be appreciated that carious changes in form and detail may be made without departing from the intended scope as defined in the appended claims.

Although the present invention is defined in the attached claims, it should be understood that the present invention can also (alternatively) be defined in accordance with the following embodiments:
1. An electronic device, comprising:
   a display for displaying information to a user;
   a storage medium for storing data and computer programming instructions; and
   a processor for executing the computer programming instructions, said computer programming instructions causing the processor to perform the following:
      exhibit a user interface for calculating a dosage of a medicament for delivery by a medicament delivery device to the user on the display, the user interface including:
      an element for entering an amount of carbohydrates to be ingested by the user,
      an element for entering a current glucose level reading,
      an element for specifying a calculated total bolus dosage, and
      an element for specifying a medicament on board adjustment value, which displays how much the medicament already delivered to the user will compensate for the carbohydrates to be ingested; and
      exhibit on the display the medicament on board adjustment value in the element for specifying a medicament on board adjustment value based on entry of a value in the element for entering an amount of carbohydrates to be ingested by the user or entry of a value in the element for entering a current glucose level reading.
2. The electronic device of embodiment 1, wherein the medicament is at least one of insulin, a glucagon-like peptide receptor-1 (GLP-1) agonist or pramlintide.
3. The electronic device of one of the preceding embodiments, wherein the user interface includes an element for displaying an adjustment to the bolus dosage due to a glucose level trend of the user.
4. The electronic device of one of the preceding embodiments, wherein the computer programing instructions cause the processor to calculate the adjustment to the bolus dosage due to the glucose level trend of the user.
5. The electronic device of one of the preceding embodiments, wherein the computer programing instructions cause the processor to calculate the medicament on board adjustment value.
6. The electronic device of one of the preceding embodiments, wherein the element for specifying the calculated total bolus dosage is editable to adjust the calculated total bolus dosage.
7. The electronic device of one of the preceding embodiments, wherein the element for entering a current glucose level reading is activatable to retrieve the current glucose level reading.
8. The electronic device of one of the preceding embodiments, wherein the electronic device is one of the medicament delivery device, a controller device for the medicament delivery device, a computing device or a wearable electronic device.
9. An electronic device, comprising:
   a display for displaying information to a user;
   a storage medium for storing data and computer programming instructions; and
   a processor for executing the computer programming instructions, said computer programming instructions causing the processor to perform the following:
      exhibit a user interface for calculating a dosage of a medicament for delivery by a medicament delivery device to the user on the display, the user interface including:
      an element for entering an amount of carbohydrates to be ingested by the user, and
      an element for specifying a calculated total bolus dosage;
      calculate an estimate of the amount of carbohydrates to be ingested by the user from data of past amounts of carbohydrate ingestion by the user; and
      prepopulate the element for entering the amount of carbohydrates to be ingested by the user with the calculated estimate of the amount of carbohydrates to be ingested by the user.
10. The electronic device of embodiment 9, wherein calculating the estimate of the amount of carbohydrates to be ingested by the user comprises calculating an average of the past amounts of carbohydrate ingestion by the user.
11. The electronic device of one of embodiments 9 or 10, wherein the calculating an average of the past amounts of carbohydrate ingestion of the user comprises calculating an average of only ones of the past amounts of carbohydrate ingestion of the user that are for a like time of day to a current time of day.
12. The electronic device of one of embodiments 9 to 11, wherein each past amount of carbohydrate ingestion of the user is associated with a type of meal, wherein the method further comprises identifying a type of a current meal to be ingested and the wherein the calculating the average of the past amounts of carbohydrates ingestion of the user comprises calculating an average of only ones of the past amounts of carbohydrate ingestion of the user that are for a like type of meal as the current meal to be ingested.
13. The electronic device of one of embodiments 9 to 12, wherein the type of meal is one of breakfast, lunch, dinner or snack.
14. The electronic device of one of embodiments 9 to 13, wherein the computer programming instructions further cause the processor to visually distinguish the prepopulated calculated estimate of the amount of carbohydrates to be ingested by the user from an amount of carbohydrates to be ingested that has been entered by the user via the element for entering the amount of carbohydrates to be ingested by the user.
15. A method performed by a processor of an electronic device, comprising:
   estimating with the processor a quantity of a medicament that will be delivered by an automated medicament delivery device over an upcoming range of time during a current day by calculating an average quantity of medicament delivered to the user by the automated medicament delivery device for the range of time on past days; and
   displaying on a display the estimated quantity as part of a user interface for specifying a bolus dosage of the medicament to be delivered by the automated medicament delivery device to assist the user.
16. The method of embodiment 15, wherein the estimated quantity is a range of quantities.
17. The method of one of embodiments 15 or 16, wherein the estimating comprises estimating a minimum quantity of medicament that will be delivered by the automated medicament delivery device over the upcoming range of time during the current day and estimating a maximum quantity of medicament that will be delivered by the automated medicament delivery device over the upcoming range of time during the current day, wherein the minimum quantity and the maximum quantity constitute the boundaries of the range of quantities.
18. The method of one of embodiments 15 to 17, wherein the minimum quantity of medicament is estimated based at least in part on latest glucose level reading, target glucose level, correction factor for the user and medicament on board.
19. The method of one of embodiments 15 to 18, wherein the minimum quantity of medicament is estimated based at least in part on latest glucose level reading, target glucose level, medicament on board and basal medicament deliveries to be delivered over the range of time.
20. The method of one of embodiments 15 to 19, wherein the medicament is at least one of insulin, a glucagon-like peptide receptor-1 (GLP-1) agonist or pramlintide.
Although the present invention is defined in the attached claims, it should be understood that the present invention can also (alternatively) be defined in accordance with the following embodiments:
1. An electronic device, comprising:
   a display for displaying information to a user;
   a storage medium for storing data and computer programming instructions; and
   a processor for executing the computer programming instructions, said computer programming instructions causing the processor to perform the following:
      exhibit a user interface for calculating a dosage of a medicament for delivery by a medicament delivery device to the user on the display, the user interface including:
         an element for entering an amount of carbohydrates to be ingested by the user,
         an element for entering a current glucose level reading,
         an element for specifying a calculated total bolus dosage, and
         an element for specifying a medicament on board adjustment value, which displays how much the medicament already delivered to the user will compensate for the carbohydrates to be ingested; and
      exhibit on the display the medicament on board adjustment value in the element for specifying a medicament on board adjustment value based on entry of a value in the element for entering an amount of carbohydrates to be ingested by the user or entry of a value in the element for entering a current glucose level reading.
2. The electronic device of embodiment 1, wherein the medicament is at least one of insulin, a glucagon-like peptide receptor-1 (GLP-1) agonist or pramlintide.
3. The electronic device of one of the preceding embodiments, wherein the user interface includes an element for displaying an adjustment to the bolus dosage due to a glucose level trend of the user.
4. The electronic device of one of the preceding embodiments, wherein the computer programing instructions cause the processor to calculate the adjustment to the bolus dosage due to the glucose level trend of the user.
5. The electronic device of one of the preceding embodiments, wherein the computer programing instructions cause the processor to calculate the medicament on board adjustment value.
6. The electronic device of one of the preceding embodiments, wherein the element for specifying the calculated total bolus dosage is editable to adjust the calculated total bolus dosage.
7. The electronic device of one of the preceding embodiments, wherein the element for entering a current glucose level reading is activatable to retrieve the current glucose level reading.
8. The electronic device of one of the preceding embodiments, wherein the electronic device is one of the medicament delivery device, a controller device for the medicament delivery device, a computing device or a wearable electronic device.
9. An electronic device, comprising:
   a display for displaying information to a user;
   a storage medium for storing data and computer programming instructions; and
   a processor for executing the computer programming instructions, said computer programming instructions causing the processor to perform the following:
      exhibit a user interface for calculating a dosage of a medicament for delivery by a medicament delivery device to the user on the display, the user interface including:
         an element for entering an amount of carbohydrates to be ingested by the user, and
         an element for specifying a calculated total bolus dosage;
      calculate an estimate of the amount of carbohydrates to be ingested by the user from data of past amounts of carbohydrate ingestion by the user; and
      prepopulate the element for entering the amount of carbohydrates to be ingested by the user with the calculated estimate of the amount of carbohydrates to be ingested by the user.
10. The electronic device of embodiment 9, wherein calculating the estimate of the amount of carbohydrates to be ingested by the user comprises calculating an average of the past amounts of carbohydrate ingestion by the user.
11. The electronic device of one of embodiments 9 or 10, wherein the calculating an average of the past amounts of carbohydrate ingestion of the user comprises calculating an average of only ones of the past amounts of carbohydrate ingestion of the user that are for a like time of day to a current time of day.
12. The electronic device of one of embodiments 9 to 11, wherein each past amount of carbohydrate ingestion of the user is associated with a type of meal, wherein the method further comprises identifying a type of a current meal to be ingested and the wherein the calculating the average of the past amounts of carbohydrates ingestion of the user comprises calculating an average of only ones of the past amounts of carbohydrate ingestion of the user that are for a like type of meal as the current meal to be ingested.
13. The electronic device of one of embodiments 9 to 12, wherein the type of meal is one of breakfast, lunch, dinner or snack.
14. A method performed by a processor of an electronic device, comprising:
   estimating with the processor a quantity of a medicament that will be delivered by an automated medicament delivery device over an upcoming range of time during a current day by calculating an average quantity of medicament delivered to the user by the automated medicament delivery device for the range of time on past days; and
   displaying on a display the estimated quantity as part of a user interface for specifying a bolus dosage of the medicament to be delivered by the automated medicament delivery device to assist the user.

## Claims

1. An electronic device, comprising:
a display (127) for displaying information to a user;
a storage medium (118) for storing data and computer programming instructions; and
a processor (119) for executing the computer programming instructions, said computer programming instructions causing the processor to perform the following:
exhibit a user interface (123) for calculating a dosage of a medicament for delivery by a medicament delivery device (102) to the user on the display (127), the user interface including:
an element (1302) for entering an amount of carbohydrates to be ingested by the user, and
an element (1318) for specifying a calculated total bolus dosage;
calculate an estimate (1304) of the amount of carbohydrates to be ingested by the user from data of past amounts of carbohydrate ingestion by the user; and
prepopulate the element (1302) for entering the amount of carbohydrates to be ingested by the user with the calculated estimate of the amount of carbohydrates to be ingested by the user,
visually distinguish the prepopulated calculated estimate of the amount of carbohydrates to be ingested by the user from an amount of carbohydrates to be ingested that has been entered by the user via the element for entering the amount of carbohydrates to be ingested by the user.

2. The electronic device of claim 1, wherein calculating the estimate of the amount of carbohydrates to be ingested by the user comprises calculating an average of the past amounts of carbohydrate ingestion by the user.

3. The electronic device of claim 1 or 2, wherein the calculating an average of the past amounts of carbohydrate ingestion of the user comprises calculating an average of only ones of the past amounts of carbohydrate ingestion of the user that are for a like time of day to a current time of day.

4. The electronic device of one of the preceding claims, wherein each past amount of carbohydrate ingestion of the user is associated with a type of meal, wherein the method further comprises identifying a type of a current meal to be ingested and the wherein the calculating the average of the past amounts of carbohydrates ingestion of the user comprises calculating an average of only ones of the past amounts of carbohydrate ingestion of the user that are for a like type of meal as the current meal to be ingested.

5. The electronic device of one of one of the preceding claims, wherein the type of meal is one of breakfast, lunch, dinner or snack.

6. The electronic device of one of one of the preceding claims, wherein the user interface permits overriding of prepopulated input values, in particular the carbohydrates value.

7. The electronic device of one of one of the preceding claims, wherein the prepopulated value is visually distinguished to identify the value as a prepopulated value that may be overridden.

8. The electronic device of one of one of the preceding claims, wherein the carbohydrates amount value (1304) is visually distinguished to indicate that it is a prepopulated value by graying the value (1304) out, or by other types of visual distinction, such as color, highlights, font choice, to identify the value is a prepopulated value.

9. The electronic device of one of one of the preceding claims, wherein the calculated estimate is displayed with a visual characteristic the indicates that the estimate may be overridden.

10. The electronic device of one of one of the preceding claims, wherein a cursor (1322) for entry of a new value can be displayed, and wherein, when the user enters a value, the value is accepted and displayed on the screen display.

11. The electronic device of one of one of the preceding claims, wherein the carbohydrates amount user interface element can be activated by the user, and the display of the estimate can be removed.

12. The electronic device of claim 11, wherein the activation comprises positioning a cursor (1322) on the calculated estimate and selecting it, or a swipe in an area (1518) bounded by the element for entering the amount of carbohydrates.

13. The electronic device of one of the preceding claims, wherein the calculated total bolus dosage is calculated using the prepopulated calculated estimate unless and until the overriding amount of carbohydrates is received, and is recalculated using the overriding amount of carbohydrates when the overriding amount is received.

14. The electronic device of one of the preceding claims, wherein the user interface further includes an element (1306) for entering a current glucose level of the user and a calculations section (1308) displaying a meal bolus value (1310), a correction bolus value (1312), a medicament on board adjustment value (1314) and a glucose trend adjustment value (1316), and wherein the calculated total bolus dosage and the calculations section are updated when the calculated estimate is removed.

15. The electronic device of one of the preceding claims, wherein the average is calculated as a sum, over a number k of days, of the amounts of carbohydrates of the meals ingested on each day, divided by the total number of meals over the k days, wherein the calculated estimate is preferably rounded down to the closest 5 gram increment, or
wherein the average is calculated using a moving sum of at least three consecutive cycles per meal, each cycle preferably being a 5 minute interval, or
wherein the average is calculated from only ones of the past amounts of carbohydrate ingestion that are for a like time of day to a current time of day.

16. The electronic device of one of the preceding claims, wherein each past amount of carbohydrate ingestion is associated with a type of meal, and the processor identifies a type of a current meal to be ingested and calculates the average from only ones of the past amounts that are for a like type of meal as the current meal,
wherein the type of meal is optionally one of breakfast, lunch, dinner or snack, or
wherein the type of meal is optionally designated based on information provided by the user or on time periods that are typical for such meals.

17. The electronic device of one of the preceding claims, wherein the visual characteristic is reverted or removed when the overriding amount of carbohydrates is received so that the overriding amount is displayed with a visual characteristic of a user-entered value.

18. The electronic device of one of the preceding claims, wherein the medicament is at least one of insulin, a glucagon-like peptide receptor-1 (GLP-1) agonist or pramlintide.

19. The electronic device of one of the preceding claims, wherein the electronic device is one of the medicament delivery device (102), a controller device for the medicament delivery device, a computing device (126), a management device or a wearable electronic device (130).
